# EUROPEAN PATENT APPLICATION

(11) **EP 4 714 334 A1**
(43) Date of publication of application: **25.03.2026**
(21) Application number: 25198722.8
(22) Date of filing: 28.08.2025
(51) Int. Cl.: A61B 3/00

(54) **HEADREST FOR EYE EXAMINATION DEVICE HAVING MODEL EYE**

(30) Priority: 19.09.2024 KR 20240126800
(71) Applicant: Huvitz Co., Ltd., Anyang-si, Gyeonggi-do 14055 (KR)
(72) Inventor: LEE, Young Woo, 14055 Anyang-si (KR)
(74) Representative: IPAZ

(57) **Abstract**

Disclosed is a headrest for an eye examination device having a model eye, in which the chin of a subject is supported when examining the eyes of the subject, and the built-in model eye is moved to and exposed at an examination position when using the model eye. The headrest for an eye examination device includes: a body (100) having a lifting space part (102) therein and positioned in front of the eye examination device; an inner body (200) having a second lifting space part (202) therein and positioned to be liftable within the lifting space part (102) of the body (100); a second inner body (300) positioned to be liftable within the second lifting space part (202); a first lifting part (400) installed on the body (100) to lift the inner body (200); a second lifting part (500) installed on the inner body (200) to lift the second inner body (300); a model eye (600) installed on the second inner body (300) so as to be lifted and exposed from the inner body (200) by the second lifting part (500); a forehead support part (700) installed on an upper side of the body (100) to position and support the forehead of a subject; and a chin support part (800) installed at an upper end of the second inner body (300) to position and support the chin of the subject.

## Description

### FIELD OF THE DISCLOSURE

The present disclosure relates to a headrest for an eye examination device, and more particularly, to a headrest for an eye examination device having a model eye, in which, when examining the eye of a subject, the chin of the subject is supported, and when using the model eye, the built-in model eye is moved to an examination position and exposed.

### BACKGROUND

In general, an eye examination device irradiates measurement light onto the cornea, iris, and retina of a subject's eye through an optical system composed of a plurality of lenses and mirrors, and forms an image of the light reflected therefrom on an image sensor, thereby converting the optical signal into an electrical signal. A calculation device calculates the electrical signal into a predetermined measurement value, and compares the calculated measurement value with predetermined reference data to measure various conditions of the subject's eye.

For example, an eye examination device is equipment for measuring the cornea, pupil diameter, corneal curvature, and refractive power of the eye, and for diagnosing cataracts, asymmetric pupils, presbyopia, and the like.

The reference data are data measured by using a model eye manufactured to simulate a human eye having standard characteristics.

Even when measuring the same examination target, the measurement value may vary depending on the specific optical system characteristics of the eye examination device used. Therefore, the reference data of the eye examination device is measured by using a model eye having standard characteristics (this is referred to as calibration of the eye examination device), and the characteristics of the actual eye being examined are measured based on the measured reference data.

Meanwhile, even if the reference data of the eye examination device is measured using a model eye at the time of manufacturing a given eye examination device and stored therein, the reference data cannot permanently serve as the reference data for the device.

For example, if deformation of the optical system occurs due to transportation of the eye examination device, thermal factors internal and external to the device, or mechanical internal stress, or if the position of the optical system changes slightly due to long-term use of the eye examination device, it is difficult to use the reference data measured during manufacturing as the reference data for the deformed eye examination device.

In this case, the model eye must be accurately positioned at the examination position of the eye examination device without tilting in the vertical or horizontal direction, and the model eye must be measured to newly generate reference data.

Conventionally, a model eye stand is used to position the model eye at the examination position. As disclosed in Korean Patent No. 10-0428476, the position of the model eye stand is adjusted so that the model eye is accurately positioned at the examination position of the eye examination device. Alternatively, the model eye may be installed in a headrest that fixes the head of the subject.

However, since the model eye is not always used, it must be stored separately when not in use, and there is a problem in that the model eye may be easily lost.

Accordingly, there is a demand for the development of a technology that prevents the loss of the model eye and enables convenient use of the model eye.

### Prior Art Literature

### (Patent Document 1) Korean Patent No. 10-0428476 (Apr. 12, 2004)

### SUMMARY OF THE DISCLOSURE

### TECHNICAL OBJECTS

An object of the present disclosure is to provide a headrest for an eye examination device having a model eye, in which, when examining the eye of a subject, the chin of the subject is supported so that the subject's eyes are positioned at an examination position, and when using the model eye, the built-in model eye is moved to the examination position and exposed.

### TECHNICAL SOLUTION

In order to achieve the above object, the present disclosure provides a headrest for an eye examination device, comprising: a body 100 having a lifting space part 102 therein and positioned in front of the eye examination device; an inner body 200 having a second lifting space part 202 therein and positioned to be liftable within the lifting space part 102 of the body 100; a second inner body 300 positioned to be liftable within the second lifting space part 202; a first lifting part 400 installed on the body 100 to lift the inner body 200; a second lifting part 500 installed on the inner body 200 to lift the second inner body 300; a model eye 600 installed on the second inner body 300 so as to be lifted and exposed from the inner body 200 by the second lifting part 500; a forehead support part 700 installed on the upper side of the body 100 to position and support the forehead of a subject; and a chin support part 800 installed at the upper end of the second inner body 300 to position and support the chin of the subject.

Preferably, the first lifting part 400 includes: a first lifting rod 410 having threads along its outer circumferential surface and rotatably installed on the body 100; a first fixing block 420 engaged with the threads of the first lifting rod 410 and installed at a lower end of the inner body 200 so as to be lifted and lowered by the rotation of the first lifting rod 410; a first lifting drive part 430 configured to rotate the first lifting rod 410; and a first guide rod 440 configured to guide the first fixing block 420 lifted and lowered by the first lifting rod 410.

Preferably, the second lifting part 500 includes: a second lifting rod 510 having threads along its outer circumferential surface and rotatably installed on the inner body 200; a second fixing block 520 engaged with the threads of the second lifting rod 510 and installed at a lower end of the second inner body 300 so as to be lifted and lowered by the rotation of the second lifting rod 510; and a second lifting drive part 530 configured to rotate the second lifting rod 510.

In addition, a model eye detachment part 610 configured to detachably install the model eye 600 on the second inner body 300 may be further included.

The model eye detachment part 610 may be a model eye installation hole 612 formed through the upper end of the second inner body 300, into which the model eye 600 is inserted.

Preferably, the inner body 200 further includes a collision prevention part 620 configured to prevent the model eye 600 from colliding with the inner body 200 when the second inner body 300 descends into the inner body 200.

Preferably, the headrest for an eye examination device according to the present disclosure further includes a controller 900 configured to control the first lifting part 400 and/or the second lifting part 500.

Preferably, the controller 900 includes: a first control mode 910 configured to lower the inner body 200 and the second inner body 300, on which the chin support part 800 is formed, so that the chin and forehead of a subject are respectively positioned on the chin support part 800 and the forehead support part 700, and the chin of the subject is seated in order to examine the eyes of the subject; and a second control mode 920 configured to control the first lifting part 400 and the second lifting part 500 to lift the inner body 200 and the second inner body 300 so that the model eye 600 is lifted to and exposed at an examination position.

### EFFECTS OF THE DISCLOSURE

According to the headrest for an eye examination device having a model eye of the present disclosure, when examining the eye of a subject, the chin of the subject is supported so that the subject's eyes are positioned at the examination position, and when using the model eye, the built-in model eye is moved to and exposed at the examination position. Therefore, the model eye can be conveniently used, and when not using the model eye, it can be stored again inside the device to prevent loss.

### BRIEF DESCRIPTION OF THE DRAWINGS

FIG. 1 is a view showing a state in which the model eye is embedded in the headrest for an eye examination device according to an embodiment of the present disclosure.
FIG. 2 is a view showing a state in which the model eye is exposed in the headrest for an eye examination device according to an embodiment of the present disclosure.
FIG. 3 is a view showing the internal structure of the headrest for an eye examination device according to an embodiment of the present disclosure.
FIG. 4 is a view showing the collision prevention part provided in the headrest for an eye examination device according to an embodiment of the present disclosure.
FIG. 5 is a view showing the controller used in the headrest for an eye examination device according to an embodiment of the present disclosure.

### DETAILED DESCRIPTION OF THE PREFERRED EMBODIMENTS

Hereinafter, the present disclosure will be described in detail with reference to the accompanying drawings.

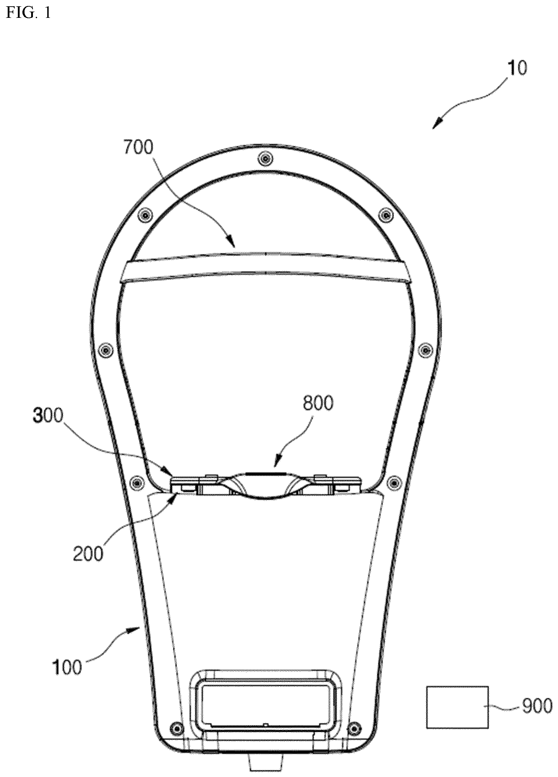

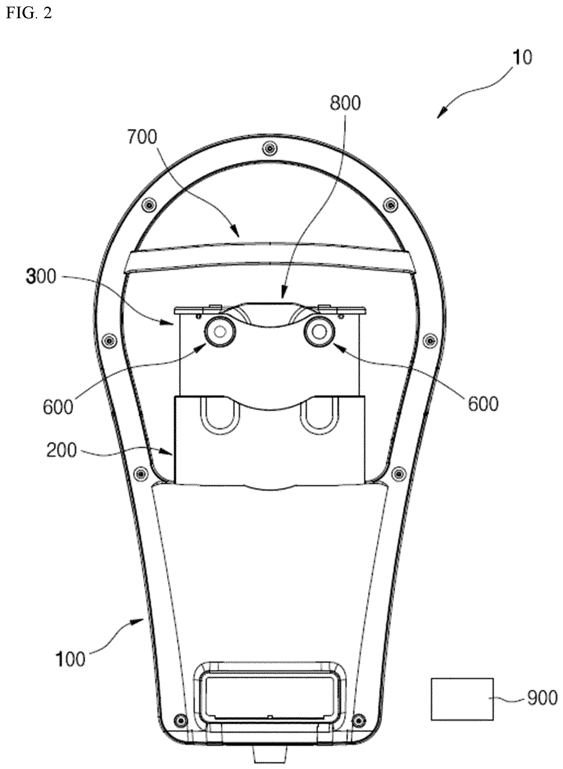

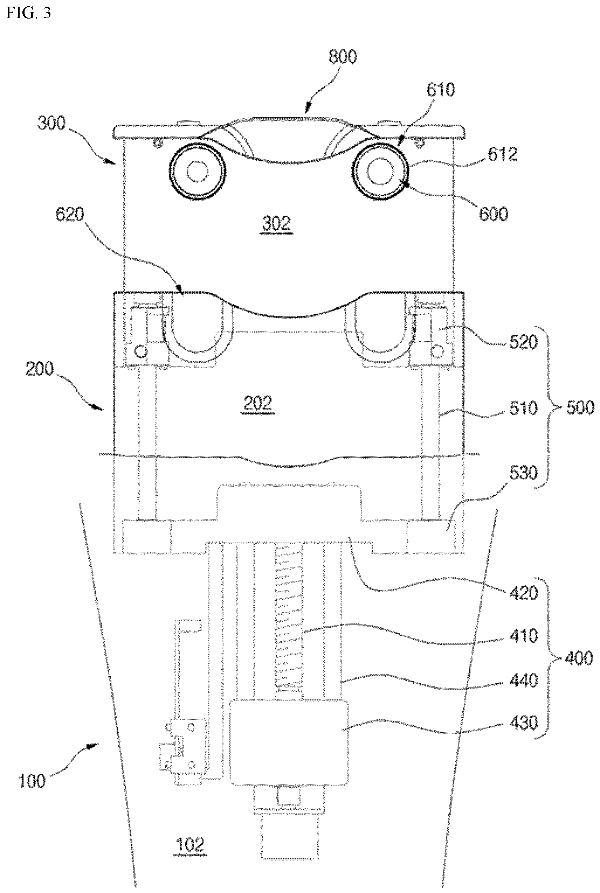

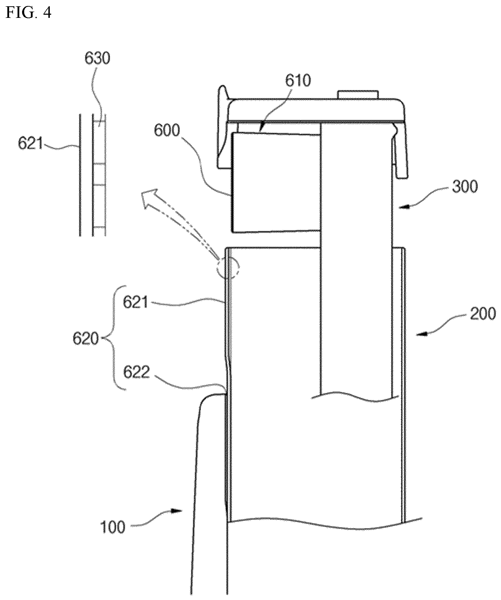

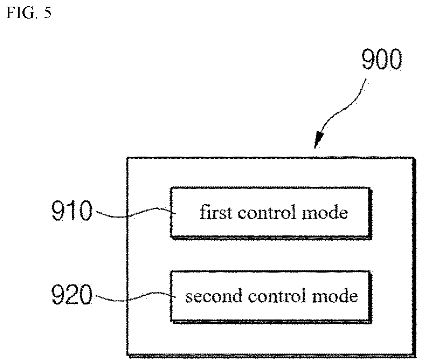

FIG. 1 is a view showing a state in which the model eye is embedded in the headrest for an eye examination device according to an embodiment of the present disclosure. FIG. 2 is a view showing a state in which the model eye is exposed in the headrest for an eye examination device according to an embodiment of the present disclosure. FIG. 3 is a view showing the internal structure of the headrest for an eye examination device according to an embodiment of the present disclosure. FIG. 4 is a view showing the collision prevention part provided in the headrest for an eye examination device according to an embodiment of the present disclosure. FIG. 5 is a view showing the controller used in the headrest for an eye examination device according to an embodiment of the present disclosure.

As shown in the drawings, the headrest 10 for an eye examination device having a model eye according to the present disclosure includes a body 100, an inner body 200, a second inner body 300, a first lifting part 400, a second lifting part 500, a model eye 600, a forehead support part 700, and a chin support part 800.

The body 100 has a lifting space part 102 therein and is positioned in front of the eye examination device.

The inner body 200 has a second lifting space part 202 therein and is positioned to be liftable within the lifting space part 102 of the body 100.

The second inner body 300 is positioned to be liftable within the second lifting space part 202 of the inner body 200.

In addition, the first lifting part 400 is installed on the body 100 and lifts the inner body 200.

The second lifting part 500 is installed on the inner body 200 and lifts the second inner body 300.

The model eye 600 is installed on the second inner body 300 so as to be lifted and exposed from the inner body 200 by the second lifting part 500.

The forehead support part 700 is installed on the upper side of the body 100 so that the forehead of the subject is positioned and supported.

The chin support part 800 is installed at the upper end of the second inner body 300 so that the chin of the subject is positioned and supported.

In an embodiment of the present disclosure, the controller 900 controls the first lifting part 400 and/or the second lifting part 500.

According to the headrest 10 for an eye examination device having a model eye, as shown in FIG. 1, in a state where the model eye is embedded in the headrest 10, the forehead of the subject may be positioned on the forehead support part 700, and the chin of the subject may be positioned on the chin support part 800, and then the eye of the subject may be examined. In addition, as shown in FIG. 2, in a state where the model eye is lifted and exposed from the inside of the headrest 10, reference data of the eye examination device may be obtained by using the model eye 600 (that is, the eye examination device may be calibrated).

As shown in FIG. 3, the first lifting part 400 includes a first lifting rod 410, a first fixing block 420, a first lifting drive part 430, and a first guide rod 440.

The first lifting rod 410 has threads along its outer circumferential surface and is rotatably installed on the body 100.

The first fixing block 420 is engaged with the threads of the first lifting rod 410 and is installed at a lower end of the inner body 200 so as to be lifted and lowered by the rotation of the first lifting rod 410.

The first lifting drive part 430 rotates the first lifting rod 410 and is, for example, a driving means such as a motor.

The first guide rod 440 guides the first fixing block 420 that is lifted and lowered by the first lifting rod 410, and guides the first fixing block 420 to move in the vertical direction and prevents it from tilting or rotating.

The second lifting part 500 includes a second lifting rod 510, a second fixing block 520, and a second lifting drive part 530.

The second lifting rod 510 has threads along its outer circumferential surface and is rotatably installed on the inner body 200.

The second fixing block 520 is engaged with the threads of the second lifting rod 510 and is installed at a lower end of the second inner body 300 so as to be lifted and lowered by the rotation of the second lifting rod 510.

The second lifting drive part 530 rotates the second lifting rod 510 and is, for example, a driving means such as a motor.

The second inner body 300 that is lifted and lowered by the second lifting part 500 may be guided by the inner body 200 that forms the inner surface of the second lifting space part 202, and thereby may be lifted and lowered in the vertical direction without tilting or twisting.

The headrest 10 for an eye examination device having a model eye may further include a model eye detachment part 610.

By means of the model eye detachment part 610, the model eye 600 may be detachably installed on the second inner body 300.

The model eye detachment part 610 may be a model eye installation hole 612 formed as a through-hole at the upper end of the second inner body 300. Since the model eye 600 is inserted into the model eye installation hole 612, other types of model eyes may be replaced and installed.

In addition, as shown in FIG. 4, the headrest 10 for an eye examination device having a model eye may further include a collision prevention part 620, if necessary.

The collision prevention part 620 prevents the model eye 600 from colliding with the inner body 200 when the second inner body 300 descends into the inner body 200.

The collision prevention part 620 includes a collision prevention protrusion 621 and a collision prevention seating groove 622.

The collision prevention protrusion 621 is formed to protrude from a corresponding portion of the inner body 200 so that a spaced gap is formed between the protrusion and the model eye 600.

The collision prevention seating groove 622 is formed on the body 100 so that the collision prevention protrusion 621 may be positioned therein.

The collision prevention part 620 may further include a model eye cleaner 630.

The model eye cleaner 630 is installed on the collision prevention protrusion 621 so as to be positioned in a spaced gap formed by the collision prevention protrusion 621. As the descending model eye 600 comes into contact with the model eye cleaner 630, dust and the like attached to the model eye 600 are wiped and removed by the model eye cleaner 630.

The model eye cleaner 630 may be configured of at least one of a plurality of brushes having a predetermined length, a scraper, or a sponge or fabric capable of containing a cleaning liquid.

As shown in FIG. 5, the controller 900 may be operated in a first control mode 910 and a second control mode 920.

The first control mode 910 is configured to lower the inner body 200 and the second inner body 300, on which the chin support part 800 is formed, so that the chin and forehead of a subject are respectively positioned on the chin support part 800 and the forehead support part 700 without using the model eye 600, and the chin of the subject is seated in order to examine the eyes of the subject.

In more detail, the controller 900 controls the first lifting part 400 and the second lifting part 500 to lower the inner body 200 and the second inner body 300 so that the chin support part 800 is positioned to receive the chin.

The second control mode 920 is for obtaining reference data of the eye examination device using the model eye 600, and the controller 900 controls the first lifting part 400 and the second lifting part 500 to lift the inner body 200 and the second inner body 300 so that the model eye 600 is lifted to and exposed at the examination position.

As shown in FIG. 2, in the second control mode 920, the controller 900 controls the second lifting part 500 to lift the second inner body 300 and expose the model eye 600.

The lifting of the inner body 200 and/or the second inner body 300 may be performed by the controller 900 driving the first lifting drive part 430 and/or the second lifting drive part 530, or, if necessary, the user may manually lift and lower the inner body 200 and/or the second inner body 300.

Although the present disclosure has been described above with reference to the accompanying drawings and exemplary embodiments, the present disclosure is not limited to the matters shown in the drawings and the above-described embodiments. In the following claims, reference numerals are included for convenience of understanding, but the scope of the claims should not be limited to the reference numerals and the contents shown in the drawings, and should be interpreted to encompass all modifications, equivalent structures, and functions of the exemplary embodiments.

Description of reference numerals:
- 10: headrest
- 100: body
- 200: inner body
- 300: second inner body
- 400: first lifting part
- 500: second lifting part
- 600: model eye
- 700: forehead support part
- 800: chin support part
- 900: controller

## Claims

1. A headrest for an eye examination device, comprising:
a body (100) having a lifting space part (102) therein and positioned in front of the eye examination device;
an inner body (200) having a second lifting space part (202) therein and positioned to be liftable within the lifting space part (102) of the body (100);
a second inner body (300) positioned to be liftable within the second lifting space part (202);
a first lifting part (400) installed on the body (100) to lift the inner body (200);
a second lifting part (500) installed on the inner body (200) to lift the second inner body (300);
a model eye (600) installed on the second inner body (300) so as to be lifted and exposed from the inner body (200) by the second lifting part (500);
a forehead support part (700) installed on an upper side of the body (100) to position and support the forehead of a subject; and
a chin support part (800) installed at an upper end of the second inner body (300) to position and support the chin of the subject.

2. The headrest for an eye examination device according to claim 1, wherein the first lifting part (400) comprises:
a first lifting rod (410) having threads along an outer circumferential surface thereof and rotatably installed on the body (100);
a first fixing block (420) engaged with the threads of the first lifting rod (410) and installed at a lower end of the inner body (200) so as to be lifted and lowered by rotation of the first lifting rod (410);
a first lifting drive part (430) configured to rotate the first lifting rod (410); and
a first guide rod (440) configured to guide the first fixing block (420) that is lifted and lowered by the first lifting rod (410).

3. The headrest for an eye examination device according to claim 1 or 2, wherein the second lifting part (500) comprises:
a second lifting rod (510) having threads along an outer circumferential surface thereof and rotatably installed on the inner body (200);
a second fixing block (520) engaged with the threads of the second lifting rod (510) and installed at a lower end of the second inner body (300) so as to be lifted and lowered by rotation of the second lifting rod (510); and
a second lifting drive part (530) configured to rotate the second lifting rod (510).

4. The headrest for an eye examination device according to any one of claims 1 to 3, wherein the inner body (200) further comprises:
a collision prevention part (620) configured to prevent the model eye (600) from colliding with the inner body (200) when the second inner body (300) descends into the inner body (200).

5. The headrest for an eye examination device according to any one of claims 1 to 4, further comprising:
a controller (900) configured to control the first lifting part (400) and/or the second lifting part (500).

6. The headrest for an eye examination device according to claim 5, wherein the controller (900) is configured to:
perform a first control mode (910) in which the chin and forehead of a subject are respectively positioned on the chin support part (800) and the forehead support part (700), and the second inner body (300) on which the chin support part (800) is formed and the inner body (200) are lowered so that the chin of the subject is seated in order to examine the eyes of the subject; and
perform a second control mode (920) in which the first lifting part (400) and the second lifting part (500) are controlled to lift the inner body (200) and the second inner body (300) so that the model eye (600) is lifted to and exposed at an examination position.
